Europäisches Patentamt

European Patent Office

Office européen des brevets

(11) Veröffentlichungsnummer: **0 281 089 B1**

(12) **EUROPÄISCHE PATENTSCHRIFT**

(45) Veröffentlichungstag der Patentschrift: **30.09.92**

(51) Int. Cl.5: **A61K 35/16**, C07K 3/18, G01N 33/36

(21) Anmeldenummer: **88103137.1**

(22) Anmeldetag: **02.03.88**

(54) **Verfahren zur Herstellung von Faktor VIII:C-Mangelplasma und ein so erhaltenes Mangelplasma.**

(30) Priorität: **06.03.87 DE 3707213**

(43) Veröffentlichungstag der Anmeldung:
**07.09.88 Patentblatt 88/36**

(45) Bekanntmachung des Hinweises auf die
Patenterteilung:
**30.09.92 Patentblatt 92/40**

(84) Benannte Vertragsstaaten:
**AT BE CH DE ES FR GB IT LI LU NL SE**

(56) Entgegenhaltungen:
**US-A- 4 361 509**

**BIOCHEMISTRY, Band 24, Nr. 16, 1985, American ChemICAL Society, Easton, PA (US);
F.ROTBLAT et al., Seiten 4294-4300.**

**CHEMICAL ABSTRACTS, Band 91, Nr. 24, 10 Dezember 1979, Columbus, OH (US);
M.FURLAN et al., Seite 365, Nr. 198832t.**

(73) Patentinhaber: **BEHRINGWERKE Aktiengesellschaft
Postfach 1140
W-3550 Marburg 1(DE)**

(72) Erfinder: **Becker Udo, Dr.
Birkenweg 2
W-3550 Marburg(DE)**
Erfinder: **Heimburger, Norbert, Prof. Dr.
Sonnenhang 10
W-3550 Marburg(DE)**
Erfinder: **Braun, Konrad
Im Streu 8
W-3557 Ebsdorfergrund 8(DE)**

(74) Vertreter: **Fischer, Hans-Jürgen, Dr.
Hoechst AG Zentrale Patentabteilung Postfach 80 03 20
W-6230 Frankturt am Main 80(DE)**

EP 0 281 089 B1

**Beschreibung**

Die Erfindung bezieht sich auf ein Verfahren zum Herstellen eines Faktor VIII:C-Mangelplasmas aus einem Ausgangsplasma unter Verwendung von Antikörpern.

Unter Faktor VIII:C-Mangelplasma versteht man ein für Gerinnungsuntersuchungen geeignetes Plasma, das frei von Faktor VII:C ist, jedoch alle anderen im Plasma normalerweise vorhandenen Gerinnungsfaktoren in im wesentlichen normaler Konzentration enthält.

Ein Faktor VIII:C-Mangelplasma ist geeignet, um in Faktor VIII:C-haltigen Flüssigkeiten, beispielsweise Blut oder Plasma, den Gehalt an Faktor VIII:C zu bestimmen.

Das Prinzip einer solchen Bestimmung besteht darin, die zu bestimmende Probe mit einem Überschuß an Faktor VIII:C-Mangelplasma zu mischen und einen Gerinnungstest bekannter Art durchzuführen. Das Ergebnis dieses Gerinnungstests hängt dann nur von dem in der zu untersuchenden Blut- oder Plasmaprobe vorhandenen Gehalt an Faktor VIII:C ab, da alle übrigen Gerinnungsfaktoren im Überschuß vorhanden und somit für die Reaktion nicht geschwindigkeitsbestimmend sind.

Als Gerinnungstest ist die partielle Thromboplastinzeit (PTT) geeignet. Hierbei wird die mit dem Mangelplasma gemischte Patientenprobe mit einem Gerinnungsaktivator versetzt und die Gerinnungszeit bestimmt. In Abwesenheit von Faktor VIII:C ist die Gerinnung des Ansatzes zeitlich verzögert, in Anwesenheit von Faktor VIII:C ist die Gerinnungszeit entsprechend der vorhandenen Faktor VIII: C-Konzentration verkürzt.

Die Empfindlichkeit der Bestimmung hängt entscheidend von der Qualität des verwendeten Mangelplasmas im Hinblick auf den Restgehalt an Faktor VIII:C im Mangelplasma ab, da die Bestimmung mit einem Überschuß an Mangelplasma durchgeführt wird und somit bereits ein geringer Anteil an Faktor VIII:C, beispielsweise 1 % Restaktivität, aus folgenden Gründen nicht mehr zu tolerieren ist: Für die Diagnose der Hämophilie A (Bluterkrankheit) ist der Konzentrationsbereich an Faktor VIII:C im Blut zwischen etwa 0,5 und 5 % sehr wichtig. Das Risiko von Hämophilie A-Patienten, unstillbare, innere Blutungen zu erleiden, steigt unterhalb von 5 % Faktor VIII:C-Aktivität sehr rasch an, so daß insbesondere in diesem kritischen Bereich eine ganz exakte Messung erforderlich ist, um beispielsweise eine Therapie mit Faktor VIII:C-Konzentrat zu steuern. Wenn nun die Restaktivität des FaktorsVIII: C in einem im Überschuß zugeführten Mangelplasma im Bereich von etwa 1 bis 5 % liegt, wird der Gerinnungstest unempfindlich, die Bezugskurve zu flach,mit der Folge, daß in dem kritischen, niedrigen Konzentrationsbereich zwischen etwa 0,5 und 5 % keine exakten Messungen durchgeführt werden können.

Ein Mangelplasma einer Qualität, wie sie gemäß den obigen Schilderungen dringend erforderlich ist, kann das Plasma von Patienten sein, die selbst an schwerer Hämophilie leiden und eine Faktor VIII:C-Aktivität aufweisen, die deutlich unter 1 % liegt. Ein derartiges Mangelplasma ist naturgemäß schwer zu beschaffen,liegt nicht in der für Routinezwecke ausreichenden Menge vor und die Blutspende von Patienten, die an dieser schweren Form der Hämophilie leiden, ist mit ethischen Problemen behaftet. In jüngerer Zeit ist man weiterhin mit dem Problem konfrontiert, daß das Plasma dieser Patienten zu einem hohen Prozentsatz mit Antikörpern gegen das Human Immunodeficiency Virus belastet ist und die Verwendung eines solchen Plasmas für diagnostische Zwecke ein Infektionsrisiko in sich birgt.

Es ist daher dringend notwendig, andere Wege der Herstellung eines geeigneten Mangelplasmas zu suchen. Es bietet sich dabei an, auf chemischen Weg die Zerstörung des Faktor VIII:C vorzunehmen, wobei die Aktivität der übrigen Gerinnnungsfaktoren erhalten bleibt. Ein Beispiel für dieses Vorgehen ist das Verfahren der Autoren Chantarangkul et al. (Brit.J.Haematol.40, 471-488, 1978:"An Artificial 'Haemophilic' Plasma for one-Stage Factor VIII Assay"). Dieses Mangelplasma weist jedoch nur eine schlechte Qualität auf, das den heutigen diagnostischen Ansprüchen nicht genügt, da auch andere Gerinnungsfaktoren vermindert sind.

Mit einem anderen Verfahren kann der Faktor VIII:C durch Antikörper entfernt werden, wobei spezifische Antikörper an ein unlösliches Trägermaterial gebunden werden und das Plasma mit diesem Trägermaterial, das den spezifischen Antikörper gebunden hat, kontaktiert wird, so daß der Faktor VIII:C aus dem Plasma entfernt wird, wodurch ein Mangelplasma hergestellt werden kann.

Bei dem bisher beschriebenen Verfahren werden Antikörper verwendet, die gegen den von Willebrand-Faktor (vWF) gerichtet sind. Da der für die Gerinnungsaktivität verantwortliche Faktor VIII:C im Plasma an den vWF gebunden vorliegt (Hoyer, L.W.: The Factor VIII Complex:Structure and Function. Blood 58, 1-13,1981), kann man auf diese Weise beide Proteine aus dem Plasma entfernen. Dieses Verfahren ist beispielsweise in einer Arbeit von Furlan et al. ("Preparation of Factor VIII Deficient Plasma by Immunadsorption", Vox Sang. 36, 342-346, 1979) beschrieben. Auch dieses Verfahren ist jedoch mit dem Nachteil behaftet, daß eine deutliche Restaktivität an Faktor VIII:C verbleibt, die auch durch mehrmaliges Wiederholen der Behandlung des Plasmas mit den genannten Antikörpern nicht beseitigt werden kann. Solcherart hergestellte Mangelplasmen sind im Handel und weisen die oben als nachteilig diskutierte flache Bezugskurve auf, die speziell im niedrigen Konzentrationsbereich des Faktors VIII:C keine exakten Messungen ermöglicht. Die genannten Mangelplasmen enthalten ferner keinen vWF mehr, was der beabsichtigten Funktion eines Faktor VIII:C-Mangelplasmas - der Simulation des Plasmas eines Hämophilie A-Patienten - noch nicht voll entspricht. Ein weiteres Verfahren zur Reinigung des Faktors VIII:C ist in Biochemistry 1985, 24, S. 4294-4300 beschrieben. Diesem Verfahren nach wird eine F VIII:C haltige lösung einer Serienchromatographie unterworfen.

Die nunmehr an sich naheliegende Lösung, das Plasma mit Antikörpern gegen Faktor VIII:Ag zu behandeln, führte bisher nicht zum Erfolg. Obwohl zahlreiche Antikörper gegen Faktor VIII:Ag bekannt und beschrieben sind, z.B. von Goodall, A.et al. ("Registry of Monoclonal Antibodies to Faktor VIII and von Willebrand Factor". Thromb. Haemostas. 54, 878-891, 1985) und diese teilweise durch Zugabe zu Plasma den Faktor VIII:C funktionell hemmen, gelang es bisher nicht, Faktor VIII:C aus Plasma zu gewinnen oder ein brauchbares Mangelplasma auf diese Weise herzustellen. Diese Situation wird auch in der Patentschrift US-PS-Re 32011 deutlich, die ein Verfahren zum Isolieren von Faktor VIII:C beschreibt, wobei an einen unlöslichen Träger gebundene Antikörper gegen vWF den Komplex aus vWF und Faktor VIII:C binden. In einem nachfolgenden Verfahrensschritt wird dann durch Elution mit einer $Ca^{++}$- haltigen Lösung der Faktor VIII:C aus dem Komplex herausgelöst. Es zeigt sich somit, daß zu einem indirekten und aufwendigen Verfahren gegriffen wird, weil der direkte Weg mit Antikörpern gegen Faktor VIII:Ag nicht zum Erfolg führt.

Es bedurfte daher dringend eines einfachen und zuverlässigen Verfahrens, mit dem die Restaktivität an Faktor VIII:C in einem Mangelplasma so weit reduziert wird, daß ein qualitativ hochwertiges Mangelplasma erhalten werden kann, mit dem Aktivitäten des Faktors VIII:C von unter 5 % exakt bestimmt werden können.

Ein derartig qualitativ hochwertiges Mangelplasma kann hergestellt werden, indem ein Ausgangsplasma nacheinander mit Antikörpern gegen von Willebrand-Faktor und gegen Faktor VIII:Ag behandelt wird.

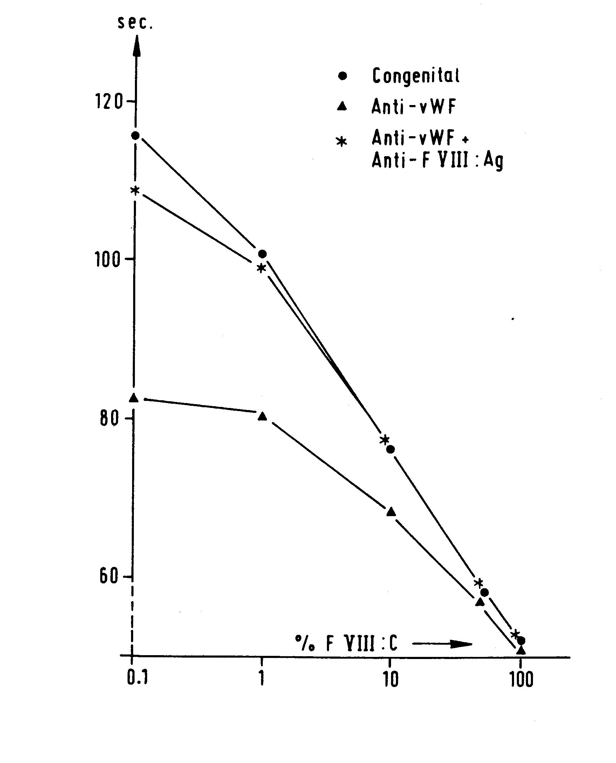

Mit der vorliegenden Erfindung wird damit ein Verfahren zum Herstellen eines qualitativ hochwertigen Mangelplasmas zur Verfügung gestellt, das den bisher bekannten Erfahrungen bei der Anwendung von Immunadsorptionsverfahren widerspricht. Es ist gelungen, durch eine kombinierte Verwendung von Antikörpern gegen von Willebrand-Faktor und von Antikörpern gegen Faktor VIII:Ag, die Restaktivität des Plasmas so zu erniedrigen, daß ein hochwertiges Mangelplasma zur Verfügung steht. Das Verfahren beinhaltet die Behandlung von Plasma mit Antikörpern gegen von Willebrand-Faktor in einem ersten Schritt und nachfolgend das Entfernen der noch verbliebenen Restaktivität mit Antikörpern gegen Faktor VIII:Ag. Jeder der genannten Schritte einzeln für sich führt nicht zum Erfolg: Nach der Behandlung des Plasmas mit Antikörpern gegen von Willebrand - Faktor verbleibt eine Restaktivität an Faktor VIII:C von etwa 1,5 %. Auch eine Wiederholung dieses Verfahrensschrittes mit bereits behandeltem Plasma führt zu keiner Verminderung der Restaktivität an Faktor VIII:C (siehe Tabelle 1). Die Behandlung des Plasmas mit Antikörpern gegen Faktor VIII:Ag alleine führt zu einem Plasma, das keinerlei nachweisbar reduzierte Aktivität an Faktor VIII:C aufweist, d.h. also, daß dieser Verfahrensschritt für sich alleine völlig unwirksam ist. Erst die kombinierte Anwendung beider Antikörper nacheinander führt zum Erfolg. Ein nach dem erfindungsgemäßen Verfahren hergestelltes Mangelplasma gleicht in seiner Bezugskurve vollständig einem kongenitalen, von einem Patienten mit hochgradiger Hämophilie A stammenden Mangelplasma (Fig.1).

Zweckmäßigerweise wird das Verfahren durchgeführt, indem die Antikörper gegen den von Willebrand-Faktor und Faktor VIII:Ag an unlösliche Trägermaterialien gebunden sind, die mit dem zu behandelnden Ausgangsplasma in Kontakt gebracht werden.

Als unlösliches Trägermaterial kann ein großporiges Gel mit einer Ausschlußgrenze von etwa > 1 Million Dalton verwendet werden.

Bei dem großporigen Gel handelt es sich bevorzugt um ein Copolymerisat aus Methacrylsäurederivaten, Pentaerythrit, PEG und Divinylbenzol.

Die verwendeten Antikörper können sowohl polyklonale und/oder monoklonale Antikörper sein, die im Handel frei erhältlich sind.

Um eine völlige Gleichheit mit einem Hämophilie-A-Plasma zu erreichen, kann der von Willebrand-Faktor, der gemäß dem erfindungsgemäßen Verfahren in einem ersten Bindungsschritt an entsprechende Antikörper entfernt wurde, nachträglich wieder zugeführt werden. Hierzu wird ein Präparat verwendet, das völlig frei von Faktor VIII:C ist, damit die Qualität des Mangelplasmas nachträglich nicht wieder vermindert wird und eine nicht mehr tolerierbare Restaktivität an Faktor VIII:C vorliegt. Derartige Präparate sind auf einfache Weise herzustellen und beispielsweise in der Veröffentlichung von Heimburger et al. ("Faktor VIII-Konzentrate - Fortschritte in der Entwicklung", Pharmazeut. Zeitung 122, 1382-1386, 1977) beschrieben.

Bevorzugt wird das Mangelplasma mit gereinigtem von Willebrand - Faktor auf 0,2-2 E/ml aufgefüllt.

Mit dem beschriebenen Verfahren läßt sich ein Faktor VIII:C-Mangelplasma herstellen, das weniger als 1 % der Norm an von Willebrand - Faktor und weniger als 0,5 % Restaktivität an Faktor VIII:C aufweist.

Nach dem Auffüllen des genannten Mangelplasmas mit von Willebrand-Faktor in einer Menge von 0,2-2 E/ml steht ein Mangelplasma zur Verfügung, das den von Willebrand - Faktor in annähernd physiologischer Konzentration enthält, jedoch nur noch eine Restaktivität des Faktors VIII:C von weniger als 0,5 %.

Die Erfindung wird durch die nachfolgenden Beispiele näher erläutert:

Beispiel 1

Antikörper gegen vWF werden an Fractogel C 75, (Merck, Darmstadt), einem Copolymerisat aus Methacrylsäurederivaten, Pentaerythrit, PEG und Divinylbenzol mittels Bromcyanmethode (Cuatrecasas, J.Biol.Chem. 245, 3059-65, 1970 ), 10 mg/ml Gelbett gebunden. Das erhaltene Antikörperadsorbens wirdin eine Säule von 2x20 cm gefüllt und mit physiologischer Kochsalzlösung äquilibriert. Über die Säule wird Citratplasma aus Humanblut, 1 l, geschickt und das Eluat aufgefangen. Die Analyse im Gerinnungstest ergibt eine Restaktivität von 1,6% (Tabelle 1). Wird mit diesem Plasma eine Bezugskurve für Faktor VIII:C erstellt, so erhält man die in Abb.1 gezeigte Kurve.

Beispiel 2

Die Durchführung des Experiments erfolgt analog Beispiel 1, jedoch wird ein monoklonaler Antikörper gegen Faktor VIII:Ag verwendet. Das Säuleneluat weist keine meßbare Reduzierung des Faktors VIII:C auf (Tabelle 1).

Beispiel 3

Das nach Beispiel 1 erhaltene Säuleneluat mit 1,6 % Restaktivität an Faktor VIII:C wird entsprechend Beispiel 2 über die Antikörpersäule mit Anti-Faktor VIII:Ag gegeben. Das Eluat weist eine Restaktivität von weniger als 0,3 % auf. Eine mit diesem Material erstellte Bezugskurve gleicht einem kongenitalen Mangelplasma eines Plasmaspenders mit schwerer Hämophilie A (Abb.1).

Beispiel 4

Das nach Beispiel 3 erhaltene Mangelplasma weist im von Willebrand-Test weniger als 1 % der Norm an vWF auf. Es wird mit gereinigtem vWF (Herstellung entsprechend Heimburger et al., Pharmazeut.Zeitung 122, 1382-1386, 1977) auf 1 E/ml aufgestockt, in silikonisierte Fläschchen zu je 1 ml abgefüllt und lyophilisiert.

EP 0 281 089 B1

## Tabelle 1

| | Adsorption von Plasma mit | | |
|---|---|---|---|
| | Anti-vWF | Anti-F VIII:Ag | Anti-vWF Anti-F VIII:Ag |
| | F VIII:C-Aktivität in % der Norm | | |
| Plasma vor Adsorption | 100 | 100 | 100 |
| Plasma nach 1. Adsorption | 1,6 | 100 | 0,13 |
| Plasma nach 2. Adsorption | 1,0 | - | - |

**Patentansprüche**

**Patentansprüche für folgende Vertragsstaaten : AT, BE, CH, DE, FR, GB, IT, LI, LU, NL, SE**

1. Verfahren zum Herstellen eines Faktor VIII:C-Mangelplasmas aus einem Ausgangsplasma unter Verwendung von Antikörpern, dadurch gekennzeichnet, daß das Ausgangsplasma nacheinander mit Antikörpern gegen von Willebrand-Faktor und gegen Faktor VIII:Ag behandelt wird und gegebenenfalls von Willebrand-Faktor zugegeben wird.

2. Verfahren nach Anspruch 1, dadurch gekennzeichnet, daß die genannten Antikörper an unlösliche Trägermaterialien gebunden sind, die mit dem Ausgangsplasma in Kontakt gebracht werden.

3. Verfahren nach Anspruch 1, dadurch gekennzeichnet, daß die Antikörper an unlösliche Trägermaterialien gebunden sind, und daß als unlösliches Trägermaterial ein großporiges Gel mit einer Auschlußgrenze von etwa größer als 1 Million Dalton verwendet wird.

4. Verfahren nach Anspruch 1, dadurch gekennzeichnet, daß die Antikörper an unlösliche Trägermaterialien gebunden sind, und daß dieses Trägermaterial ein Copolymerisat aus Methacrylsäurederivaten, Pentaerythrit, PEG und Divinylbenzol ist.

5. Verfahren nach Anspruch 1, dadurch gekennzeichnet, daß als Antikörper polyklonale und/oder monoklonale Antikörper verwendet werden.

6. Verfahren nach Anspruch 1, dadurch gekennzeichnet, daß nach der Behandlung des Plasmas mit den genannten Antikörpern der von Willebrand-Faktor in einer Menge zugeführt wird, die derjenigen im unbehandelten Ausgangsplasma entspricht.

7. Verfahren nach Anspruch 1, dadurch gekennzeichnet, daß der von Willebrand-Faktor in einer Menge von 0,2-2 E/ml zugeführt wird.

8. Faktor VIII:C-Mangelplasma, dadurch gekennzeichnet, daß es weniger als 1 % der Norm an von

5

Willebrand-Faktor und weniger als 0,5 % Restaktivität an Faktor VIII:C aufweist.

9. Mangelplasma erhältlich nach Anspruch 1, dadurch gekennzeichnet, daß der von Willebrand-Faktor in einer Menge von 0,2-2 E/ml in dem Mangelplasma enthalten ist.

**Patentansprüche für folgenden Vertragsstaat : ES**

1. Verfahren zum Herstellen eines Faktor VIII:C-Mangelplasmas aus einem Ausgangsplasma unter Verwendung von Antikörpern, dadurch gekennzeichnet, daß das Ausgangsplasma nacheinander mit Antikörpern gegen von Willebrand-Faktor und gegen Faktor VIII-Ag behandelt wird und gegebenenfalls von Willebrand-Faktor zugegeben wird.

2. Verfahren nach Anspruch 1, dadurch gekennzeichnet, daß die genannten Antikörper an unlösliche Trägermaterialien gebunden sind, die mit dem Ausgangsplasma in Kontakt gebracht werden.

3. Verfahren nach Anspruch 1, dadurch gekennzeichnet, daß die Antikörper an unlösliche Trägermaterialien gebunden sind, und daß als unlösliches Trägermaterial ein großporiges Gel mit einer Auschlußgrenze von etwa größer als 1 Million Dalton verwendet wird.

4. Verfahren nach Anspruch 1, dadurch gekennzeichnet, daß die Antikörper an unlösliche Trägermaterialien gebunden sind, und daß dieses Trägermaterial ein Copolymerisat aus Methacrylsäurederivaten, Pentaerythrit, PEG und Divinylbenzol ist.

5. Verfahren nach Anspruch 1, dadurch gekennzeichnet, daß als Antikörper polyklonale und/oder monoklonale Antikörper verwendet werden.

6. Verfahren nach Anspruch 1, dadurch gekennzeichnet, daß nach der Behandlung des Plasmas mit den genannten Antikörpern der von Willebrand-Faktor in einer Menge zugeführt wird, die derjenigen im unbehandelten Ausgangsplasma entspricht.

7. Verfahren nach Anspruch 1, dadurch gekennzeichnet, daß der von Willebrand-Faktor in einer Menge von 0,2-2 E/ml zugeführt wird.

**Claims**

**Claims for the following Contracting States : AT, BE, CH, DE, FR, GB, IT, LI, LU, NL, SE**

1. A process for the preparation of a factor VIII:C-deficient plasma from a starting plasma using antibodies, which comprises treating the starting plasma successively with antibodies against von Willebrand factor and against factor VIII:Ag and, where appropriate, adding von Willebrand factor.

2. The process as claimed in claim 1, wherein the said antibodies are bound to insoluble carriers which are contacted with the starting plasma.

3. The process as claimed in claim 1, wherein the antibodies are bound to insoluble carriers, and wherein a wide-pore gel with an exclusion limit of about over 1 million dalton is used as insoluble carrier.

4. The process as claimed in claim 1, wherein the antibodies are bound to insoluble carriers, and wherein this' carrier is a copolymer of methacrylic acid derivatives, pentaerythritol, PEG and divinylbenzene.

5. The process as claimed in claim 1, wherein polyclonal and/or monoclonal antibodies are used as antibodies.

6. The process as claimed in claim 1, wherein, after the treatment of plasma with the said antibodies, the von Willebrand factor is added in an amount which corresponds to that in the untreated starting plasma.

7. The process as claimed in claim 1, wherein the von Willebrand factor is added in an amount of 0.2-2 U/ml.

EP 0 281 089 B1

8. A factor VIII:C-deficient plasma which contains less than 1 % of the normal level of von Willebrand factor and less than 0.5 % residual activity of factor VIII:C.

9. A deficient plasma obtainable as claimed in claim 1, which contains von Willebrand factor in an amount of 0.2-2 U/ml in the deficient plasma.

**Claims for the following Contracting State : ES**

1. A process for the preparation of a factor VIII:C-deficient plasma from a starting plasma using antibodies, which comprises treating the starting plasma successively with antibodies against von Willebrand factor and against factor VIII:Ag and, where appropriate, adding von Willebrand factor.

2. The process as claimed in claim 1, wherein the said antibodies are bound to insoluble carriers which are contacted with the starting plasma.

3. The process as claimed in claim 1, wherein the antibodies are bound to insoluble carriers, and wherein a wide-pore gel with an exclusion limit of about over 1 million dalton is used as insoluble carrier.

4. The process as claimed in claim 1, wherein the antibodies are bound to insoluble carriers, and wherein this carrier is a copolymer of methacrylic acid derivatives, pentaerythritol, PEG and divinylbenzene.

5. The process as claimed in claim 1, wherein polyclonal and/or monoclonal antibodies are used as antibodies.

6. The process as claimed in claim 1, wherein, after the treatment of plasma with the said antibodies, the von Willebrand factor is added in an amount which corresponds to that in the untreated starting plasma.

7. The process as claimed in claim 1, wherein the von Willebrand factor is added in an amount of 0.2-2 U/ml.

**Revendications**
**Revendications pour les Etats contractants suivants : AT, BE, CH, DE, FR, GB, IT, LI, LU, NL, SE**

1. Procédé pour la préparation d'un plasma déficient en facteur VIII:C, à partir d'un plasma de départ, avec utilisation d'anticorps, caractérisé en ce que l'on traite le plasma de départ successivement par des anticorps dirigés contre le facteur de von Willebrand et contre le facteur VIII:Ag, et éventuellement on y ajoute du facteur de von Willebrand.

2. Procédé selon la revendication 1, caractérisé en ce que lesdits anticorps sont fixés sur des matériaux de support insolubles qui sont mis en contact avec le plasma de départ.

3. Procédé selon la revendication 1, caractérisé en ce que les anticorps sont fixés sur des matériaux de support insolubles, et en ce que l'on utilise, en tant que matériau de support insoluble, un gel à pores de grande taille, ayant une limite d'exclusion d'environ >1 000 000 d.

4. Procédé selon la revendication 1, caractérisé en ce que les anticorps sont fixés sur des matériaux de support insolubles et en ce que ce matériau de support est un copolymère obtenu à partir de dérivés de l'acide méthacrylique, de pentaérythritol, de PEG et de divinylbenzène.

5. Procédé selon la revendication 1, caractérisé en ce que l'on utilise comme anticorps des anticorps polyclonaux et/ou monoclonaux.

6. Procédé selon la revendication 1, caractérisé en ce que, après le traitement du plasma par lesdits anticorps, on introduit le facteur de von Willebrand en une quantité qui correspond à celle présente dans le plasma de départ non traité.

7. Procédé selon la revendication 1, caractérisé en ce que l'on introduit le facteur de von Willebrand en une quantité de 0,2-2 U/ml.

7

EP 0 281 089 B1

**8.** Plasma déficient en facteur VIII:C, caractérisé en ce qu'il présente moins de 1 % de la teneur normale en facteur de von Willebrand et moins de 0,5 % d'activité résiduelle du facteur VIII:C.

**9.** Plasma déficient préparable selon la revendication 1, caractérisé en ce que le facteur de von Willebrand est contenu en une quantité de 0,2-2 U/ml dans le plasma déficient.

**Revendications pour l'Etat contractant suivant : ES**

**1.** Procédé pour la préparation d'un plasma déficient en facteur VIII:C, à partir d'un plasma de départ, avec utilisation d'anticorps, caractérisé en ce que l'on traite le plasma de départ successivement par des anticorps dirigés contre le facteur de von Willebrand et contre le facteur VIII:Ag, et éventuellement on y ajoute du facteur de von Willebrand.

**2.** Procédé selon la revendication 1, caractérisé en ce que lesdits anticorps sont fixés sur des matériaux de support insolubles qui sont mis en contact avec le plasma de départ.

**3.** Procédé selon la revendication 1, caractérisé en ce que les anticorps sont fixés sur des matériaux de support insolubles, et en ce que l'on utilise, en tant que matériau de support insoluble, un gel à pores de grande taille, ayant une limite d'exclusion d'environ >1 000 000 d.

**4.** Procédé selon la revendication 1, caractérisé en ce que les anticorps sont fixés sur des matériaux de support insolubles et en ce que ce matériau de support est un copolymère obtenu à partir de dérivés de l'acide méthacrylique, de pentaérythritol, de PEG et de divinylbenzène.

**5.** Procédé selon la revendication 1, caractérisé en ce que l'on utilise comme anticorps des anticorps polyclonaux et/ou monoclonaux.

**6.** Procédé selon la revendication 1, caractérisé en ce que, après le traitement du plasma par lesdits anticorps, on introduit le facteur de von Willebrand en une quantité qui correspond à celle présente dans le plasma de départ non traité.

**7.** Procédé selon la revendication 1, caractérisé en ce que l'on introduit le facteur de von Willebrand en une quantité de 0,2-2 U/ml.

8